# DEMANDE DE BREVET EUROPEEN

(11) **EP 0 564 747 A1**
(43) Date de publication de la demande: **13.10.1993**
(21) Numéro de dépôt: 92400992.1
(22) Date de dépôt: 09.04.1992
(51) Int. Cl.: A61M 29/02

(54) **Cathéter pour la dilatation des artères**

(71) Demandeur: BALT, société anonyme, F-95160 Montmorency (FR)
(72) Inventeur: Plowiecki, Léopold, F-95160 Montmorency (FR); Starck, Erhard, 6242 Kronberg/Schonberg (DE)
(74) Mandataire: Chambon, Gérard

(57) **Abrégé**

L'invention concerne un cathéter pour la dilatation des artères pourvu d'un canal (1) unique dans lequel est logé un guide flexible (4) et qui permet en outre le passage d'un fluide pour le gonflage d'un ballonnet (3) qui est fixé à l'extrémité distale dudit cathéter et qui est pourvu d'un orifice distal (7) ménagé du côté opposé à son orifice de gonflage (6) pour le passage de l'extrémité dudit guide (4)
Le cathéter selon l'invention est remarquable en ce que le guide (4) est monté librement dans le canal du cathéter et en ce qu'il est pourvu de manière solidaire d'un obturateur (8) ayant un diamètre légèrement supérieur à celui de l'orifice distal (7) du ballonnet (3), de manière telle que ledit obturateur puisse venir à volonté obturer ou non ledit orifice en déplaçant en translation le guide (4) afin de permettre, le gonflage dudit ballonnet dans la lumière artérielle à dilater ou respectivement une injection de produit en aval du ballonnet.

L'invention est notamment destinée à la dilatation des artères des jambes et des artères coronariennes.

## Description

L'invention concerne un cathéter pour la dilatation des artères, pourvu d'un guide flexible et d'un ballonnet gonflable fixé à l'extrémité distale dudit cathéter.

Pour traiter les sténoses artérielles d'origine athéromateuses ou spasmodiques, il est connu d'avoir recours à des procédés de dilatation.

Pour faire ce type d'intervention, on utilise généralement un cathéter muni à son extrémité distale d'un ballonnet que l'on gonfle dans la sténose au moyen d'un fluide, tel qu'un produit de contraste.

L'expérience montre que pour positionner correctement le ballonnet dans l'artère ou le vaisseau à dilater, il est nécessaire d'utiliser un cathéter muni d'un guide.

En cas de rétrécissement de la lumière d'un vaisseau, il faut en effet pouvoir bénéficier d'un guidage supplémentaire au simple guidage par le flux artériel, et donc d'une certaine rigidité de l'ensemble pour la mise en place.

C'est pourquoi, il est connu d'utiliser un cathéter à deux canaux dont l'un est réservé au passage du guide, sous forme d'un fil flexible, et l'autre, au passage de l'agent de gonflage du ballonnet.

Ce type de cathéter présente notamment le grave inconvénient de nécessiter deux canaux d'où un diamètre extérieur du cathéter important et parfois rédhibitoire pour certaines applications.

Il a aussi été imaginé d'utiliser un cathéter pourvu d'un canal unique dans lequel est logé le guide flexible et qui permet en outre le passage du fluide de gonflage du ballonnet, lequel ballonnet est fixé à l'extrémité distale dudit cathéter. Le guide traverse alors complètement le ballonnet auquel il est en outre assujetti, de manière telle que le guide est fixe par rapport au cathéter et au ballonnet.

Ce type de cathéter présente notamment l'inconvénient d'une mise en place délicate du fait que l'on ne peut pas agir sur le guide par translation ou rotation de manière indépendante au cathéter lui-même.

Pour palier ces inconvénients, l'invention propose un cathéter du genre du dernier type cité, c'est-à-dire pourvu d'un canal unique dans lequel est logé un guide flexible et qui permet en outre le passage d'un fluide pour le gonflage d'un ballonnet qui est fixé à l'extrémité distale dudit cathéter et qui est pourvu d'un orifice distal ménagé du côté opposé à son orifice de gonflage pour le passage de l'extrémité dudit guide, mais qui est remarquable en ce que le guide est monté librement dans le canal du cathéter et en ce qu'il est pourvu de manière solidaire d'un obturateur ayant un diamètre légèrement supérieur à celui de l'orifice distal du ballonnet, de manière telle que ledit obturateur puisse venir à volonté obturer ou non ledit orifice en déplaçant en translation le guide afin de permettre, le gonflage dudit ballonnet dans la lumière artérielle à dilater ou respectivement une injection de produit en aval du ballonnet.

De la sorte, il est clair que le cathéter est limité à un seul canal et que la manipulation du guide reste entière. Ainsi, la progression dans l'artère, indépendamment de l'action du flux artériel, est assurée par le guide qui permet en outre un contrôle du centrage et de la stabilité du ballonnet. L'un des aspects tout à fait inventif consiste à avoir imaginé que l'orifice distal du ballonnet pouvait être obturé pendant la dilatation par un élément solidaire du guide lui-même, alors que celui-ci était monté libre dans le ballonnet.

Par exemple, l'obturateur est constitué par une bague fixée sur le guide et de préférence, il est fabriqué à partir d'une matière opaque aux rayons X.

Le ballonnet quant à lui est, par exemple, fabriqué à partir d'un polymère.

Toutes sortes de cathéters et de guides sont évidemment envisageables, mais un mode de réalisation préféré est remarquable en ce que le guide est formé au moins en partie par un enroulement hélicoïdal à l'intérieur duquel est disposé au moins un fil.

L'invention sera bien comprise à la lecture de la description qui va suivre en référence aux dessins annexés dans lesquels:
- la figure 1 montre schématiquement en coupe un cathéter selon l'invention, l'obturateur étant en position ouverte pour une injection,
- la figure 2 montre schématiquement le cathéter de la figure 1 en position d'obturation pour la dilatation,
- les figures 3 et 4 sont des vues agrandies des ballonnets montrés respectivement sur les figures 1 et 2.

Comme le montrent les dessins, le cathéter selon l'invention comporte une partie tubulaire 1 formant un canal unique. A l'extrémité proximale dudit cathéter est prévu un raccord 2 dit en "Y", tandis qu'à l'extrémité distale est fixé un ballonnet 3.

Le raccord 2 comporte un conduit axial 2a et un conduit ramifié 2b. Un guide souple 4 est aménagé pour coulisser et tourner librement dans la partie tubulaire 1 et de manière étanche dans le conduit axial 2a. L'extrémité proximale du guide 4 est pourvue d'un moyen 5 de préhension et de manoeuvre en translation et/ou en rotation du guide 4.

Le conduit 2b est prévu pour l'injection d'un fluide de gonflage du ballonnet sous forme par exemple d'un produit de contraste.

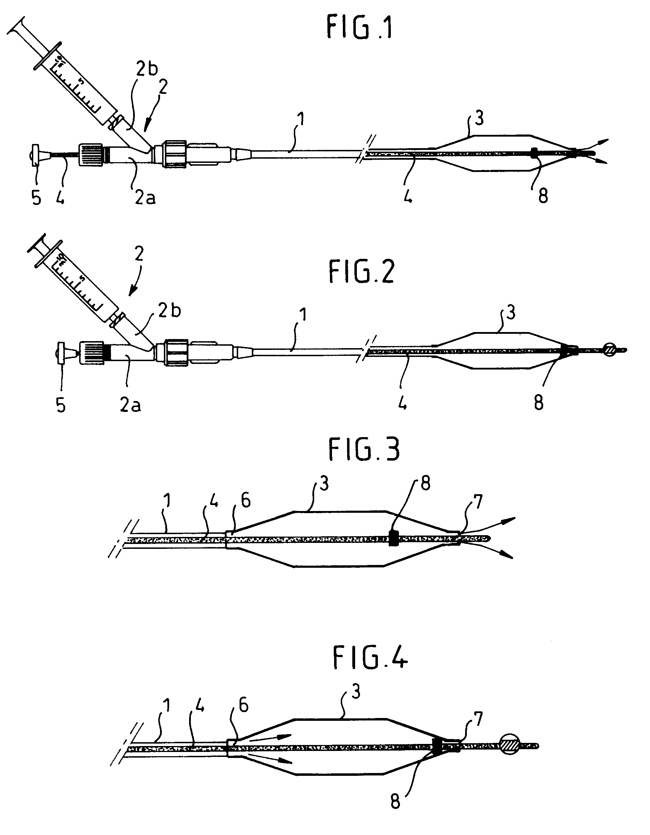

Il est clair en effet que le ballonnet 3 est fixé à l'extrémité de la partie tubulaire 1 en laissant un orifice libre de gonflage 6, comme le montrent bien les figures 3 et 4.

On peut voir en outre sur les dessins que l'extrémité du guide 4 traverse entièrement le ballonnet 3.

En effet, ce dernier comporte selon l'invention à son extrémité distale, c'est-à-dire opposée à son orifice de gonflage 6, un autre orifice, référencé en 7 sur les figures 3 et 4, et au travers duquel le guide 4 peut passer.

En outre, on peut voir aussi sur les dessins, que le guide 4 est pourvu dans sa partie située dans le ballonnet 3, d'un élément dénommé ci-après obturateur 8.

L'obturateur 8 qui est fixé de manière solidaire sur le guide 4 est constitué, par exemple, par une bague dont le diamètre est légèrement plus grand que celui de l'orifice 7 afin de jouer son rôle (mais un peu plus petit que l'orifice 6 pour le montage). On crée ainsi un véritable guide obturateur.

Avantageusement, la bague formant l'obturateur est opaque aux rayons X afin de permettre d'en visualiser la progression.

L'utilisation et le fonctionnement d'un cathéter selon l'invention sont parfaitement clairs.

On positionne et on introduit le cathéter avec son guide 4 dans une artère. Tant que l'obturateur 8 est dans la position représentée sur les figures 1 et 3, il est toujours possible d'injecter du sérum physiologique ou du produit de contraste en aval du ballonnet 3 au moyen du conduit 2b représenté sur les figures 1 et 2, lequel conduit est en outre équipé ici d'une seringue.

Lorsque le cathéter arrive à l'endroit désiré pour la dilation, on pousse le guide 4 en le faisant franchir l'orifice 7 pour le faire dépasser dudit ballonnet 3 dans ledit vaisseau rétréci et jusqu'à ce que l'obturateur 8 vienne obturer l'orifice 7 (figures 2 et 4).

Il suffit alors de gonfler le ballonnet 3 en injectant un liquide par ledit conduit 2b. Après dilatation, le dégonflage du ballonnet s'effectue par simple aspiration.

Un tel système utilisant, par exemple, un ballonnet en polymère permet de résister à des pressions importantes.

Pour la partie tubulaire 1 on pourra utiliser les cathéters à souplesse progressive déjà connus et pour le guide 4, l'inventeur préconise d'utiliser un fil qui est gainé par un enroulement hélicoïdal comme le montre la partie agrandie de la figure 4.

L'invention sert à dilater tous les vaisseaux mais plus particulièrement encore, les artères des jambes ainsi que les artères coronaires pour angioplastie coronarienne.

## Revendications

1. Cathéter pour la dilatation des artères pourvu d'un canal (1) unique dans lequel est logé un guide flexible (4) et qui permet en outre le passage d'un fluide pour le gonflage d'un ballonnet (3) qui est fixé à l'extrémité distale dudit cathéter et qui est pourvu d'un orifice distal (7) ménagé du côté opposé à son orifice de gonflage (6) pour le passage de l'extrémité dudit guide (4), caractérisé en ce que le guide (4) est monté librement dans le canal du cathéter et en ce qu'il est pourvu de manière solidaire d'un obturateur (8) ayant un diamètre légèrement supérieur à celui de l'orifice distal (7) du ballonnet (3), de manière telle que ledit obturateur puisse venir à volonté obturer ou non ledit orifice en déplaçant en translation le guide (4) afin de permettre, le gonflage dudit ballonnet dans la lumière artérielle à dilater ou respectivement une injection de produit en aval du ballonnet.

2. Cathéter selon la revendication 1, caractérisé en ce que l'obturateur est constitué par une bague (8) fixée sur le guide (4).

3. Cathéter selon l'une des revendications 1 et 2, caractérisé en ce que l'obturateur est fabriqué à partir d'une matière opaque aux rayons X.

4. Cathéter selon l'une des revendications 1 à 3, caractérisé en ce que le ballonnet est fabriqué à partir d'un polymère.

5. Cathéter selon l'une des revendications 1 à 4, caractérisé en ce que le guide (4) est formé au moins en partie par un enroulement hélicoïdal à l'intérieur duquel est disposé au moins un fil.
